# EUROPEAN PATENT SPECIFICATION

(11) **EP 1 545 304 B1**
(45) Date of publication and mention of the grant of the patent: **12.03.2008**
(21) Application number: 03799227.8
(22) Date of filing: 26.09.2003
(51) Int. Cl.: A61B 5/06, A61M 5/42

(54) **DETECTION OF IMPLANTED INJECTION PORT**
NACHWEIS EINES IMPLANTIERTEN INJEKTIONSPORTS
DETECTION D'UN ORIFICE D'INJECTION IMPLANTE

(30) Priority: 01.10.2002 US 260546
(43) Date of publication of application: 29.06.2005
(60) Divisional application: 07020884.8
(73) Proprietor: Potencia Medical AG, 6341 Baar (CH)
(72) Inventor: FORSELL, Peter, CH-6300 Zug (CH)
(74) Representative: Klunker . Schmitt-Nilson . Hirsch
(86) International application number: PCT/SE2003/001503
(87) International publication number: WO 2004/030536

(56) References cited:
- WO-A1-96/08999
- WO-A1-96/32060
- US-A- 5 622 169
- US-B1- 6 305 381

## Description

The present invention is defined in claim 1 and relates to an apparatus for detecting an injection port subcutaneously implanted in a patient. The invention also relates to an artificial sphincter apparatus comprising an artificial sphincter, and injection port connected thereto, and said injection port detecting apparatus.

It is important to locate the position of an injection port connected to a hydraulically operable surgical implant in a patient to be able to accurately inject a needle of a syringe through the membrane of the injection port (or simply for the purpose of locating the exact position of the injection port, or alternatively locating the membrane of the injection port), for supplying hydraulic fluid to or withdrawing hydraulic fluid from the implant. Such an injection port is typically arranged in connection (via a conduit) to a hydraulically adjustable implant, for example a food intake restriction device, implanted inside an obese patient.

Currently, a nurse or doctor locates an implanted injection port by simply feeling with the fingers on the patient's skin to find out where the injection port is situated. However; the nurse or doctor cannot know exactly where the injection needle should penetrate the skin, in order to penetrate the centre of the membrane of the injection port.

US-B1-6 305 381 discloses a system for locating implantable medical devices, which may be a drug infusion device having a drug reservoir. The drug reservoir has a septum through which a needle can be inserted to replenish the reservoir with drugs. In order to locate the medical device with a high degree of precision, a signal processing system is provided which is capable of determining an operating distance with the implanted device as well as an alignment between the septum of the implanted drug reservoir and a guide for a needle to be inserted through the septum. More particularly, an implanted antenna coil emits energy which is sensed with an antenna array external to the patient and comprising three antennas equally spaced from each other with the needle guide located concentrically thereto. When an equal amount of energy is present in each of the antennas of the antenna array and if each such ducted energy is greater than a predetermined minimum, then the needle guide in the antenna array is aligned with the implant coil and, thus, with the septum of the drug reservoir at a desired distance thereof.

The object of the present invention is to provide an inexpensive apparatus and methods for accurately detecting an injection port subcutaneously implanted in a patient to enable an injection needle to safely penetrate the patient's skin directly into the centre of the injection port.

This object is obtained by an apparatus of the kind stated initially, comprising a magnetic device in the form of a permanent ring magnet adapted to emit a local magnetic field, and a magnetic detector adapted to detect the local magnetic field emitted by the magnetic device. The magnetic detector is designed to be subcutaneously implanted and the permanent ring magnet is movable along the patient's body to establish the injection position at the patient's skin in front of the implanted injection port where the local magnetic field emitted by the ring magnet is detected by the magnetic detector.

Thus, the accurate injection position on the patient's skin in front of the injection port, which is hidden behind the skin, can be established using the apparatus of the present invention. With an injection needle placed in this injection position, it is an easy task to properly and safely insert the injection needle through the patient's skin directly into the injection port substantially in the centre thereof. The present invention is particularly advantageous to practise in obese people where an implanted injection port can be difficult to manually locate.

Generally, the magnetic detector includes a semiconductor circuit, preferably in the form of at least one Hall-element. By using one or more Hall-elements, a.special type of semiconductor known in the art, it is easy to locate the centre of the magnetic field emitted by the magnetic device. The magnetic detector suitably comprises several Hall-elements grouped around a central point in a triangular or square configuration. For example, three Hall-elements may be arranged at the corners of an equilateral triangle. An important advantage is that the Hall-elements are able to detect even a weak magnetic field emitted from the magnetic device.

In accordance with the invention, the magnetic detector is designed to be subcutaneously implanted in the patient at the implanted injection port, and the magnetic device, i.e. the permanent ring magnet, is adapted to emit the local magnetic field through the patient's skin from outside the patient's body and is movable externally along the patient's body to establish the injection position where the local magnetic field is detected by the implanted magnetic detector. By making the movable magnetic device in the form of a ring is, it is suitably adapted to establish the injection position in front of the subcutaneously implanted injection port. In its simplest form, the implanted magnetic detector may be adapted to emit a sound when detecting the local magnetic field. In a more sophisticated form, a sender may be implantable in the patient's body and be capable of sending information about the magnetic detector to outside the patient's body, as the implanted magnetic detector detects the local magnetic field emitted by the movable magnetic device from outside the patient's body. For example, the implanted sender may send RF signals that inform when the implanted detector detects the local magnetic field, whereby an accurate injection position at the patient's skin can be established. The accurate injection position may be directly or indirectly correlated to the intensity of magnetism detected by the magnetic detector.

The present invention can be advantageously used in a method of detecting a wireless injection port subcutaneously implanted in a patient. The method comprises providing permanent ring magnet capable of emitting a local magnetic field through the patient's skin, providing a magnetic detector adapted to detect the local magnetic field emitted by the magnetic device, subcutaneously implanting the magnetic detector in the patient at the implanted injection port, moving the ring magnet externally along the patient's body, and establishing an injection position at the patient's skin where the local magnetic field emitted by the ring magnet is detected by the magnetic detector. Then, an injection needle can be placed in the injection position where the local magnetic field has been detected to accurately insert the needle through the ring magnet and the patient's skin directly into the injection port.

In accordance with the method, the magnetic detector is subcutaneously implanted, the ring magnet is moved externally along the patient's body, and the injection position is established at the patient's skin where the local magnetic field emitted by the moving ring magnet is detected by the implanted magnetic detector. When practising the method it may further comprise implanting a sender and using the sender to send information to outside the patient's body confirming when the implanted magnetic detector detects the local magnetic field emitted by the moving ring magnet.

When practising the above detection method a semiconductor circuit, preferably comprising at least one Hall-element, may be used as the magnetic detector.

The invention is described in more detail in the following with reference to the accompanying drawings, in which
Figure 1 shows a connection diagram for a magnetic detector of the apparatus according to the present invention,
Figure 2 schematically illustrates in a diagram the output of the magnetic detector positioned in front of a magnetic device of the apparatus of the invention.
Figure 3 is a schematic view of arrangement where the magnetic detector is subcutaneously implanted in the patient and the magnetic device is movable externally along the patient's body,
Figure 4 is a schematic view of a hydraulically adjustable constriction device designed for treating reflux disease, urine incontinence, anal incontinence or obesity, and
Figure 5 illustrates an embodiment according to the present invention using Hall-elements as the magnetic detecting device.

Referring to the drawing figures, like reference numerals designate identical or corresponding elements throughout the several figures.

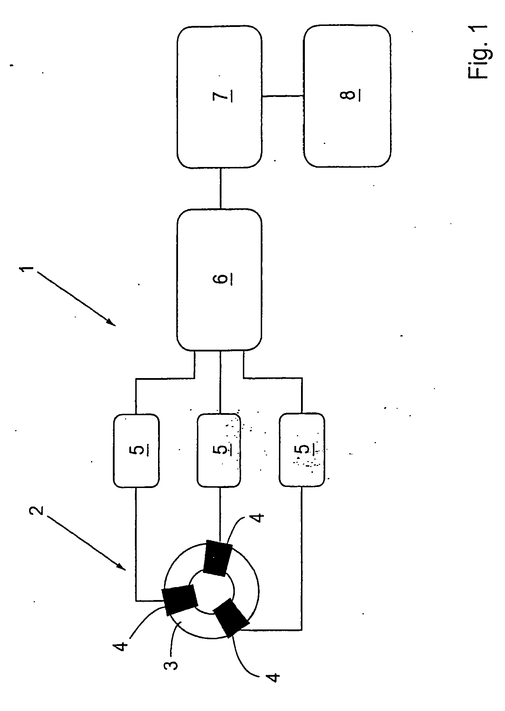

Figure 1 shows a connection circuit 1 for a magnetic detector 2 of the apparatus according to the present invention. A magnetic device in the form of a permanent ring-magnet 3 is movable externally the patient's body. Located inside the body and positioned in front of the implanted ring-magnet 3 is magnetic detector 2, which includes three linear magnetic field sensors 4 grouped in a triangular configuration. Each sensor 4 includes a semiconductor circuit such as a Hall-element or the like. Sensors 4 are connected to signal-conditioning amplifiers 5, which in turn, are connected to an A/D-converter 6. A microprocessor 7 is connected to A/D-converter 6. To visually display the output signals of sensors 4, a display-device 8 is connected to microprocessor 7.

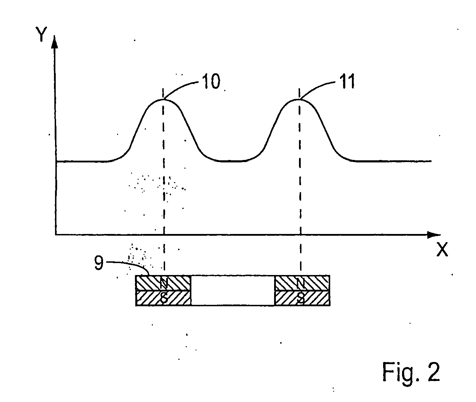

The graph shown in Fig. 2 illustrates, in principle, how the information obtained by detector 2 can be presented. On the X-axis in the graph is the position of detector 2 relative to the magnetic device. On the Y-axis is the combined output of sensors 4 of detector 2. Thus, the graph of Fig. 2 shows the position "X" of detector 2 relative to the magnetic device as a function of detector 2's output "Y": To illustrate this method of detecting, a magnetic device in the form of a ring-magnet 9 is shown relative to the graph of Figure 2. Ring-magnet 9 is shown in cross-section to show the positions of its magnetic north pole N and south pole S, respectively. Fig. 2 depicts the case where ring magnet 9 has been centred in front of magnet detector 2 (not shown Fig. 2) and where all of the sensors 4 produce a maximum output, which is shown as peaks 10,11 in the graph of Fig. 2. Sensors 4 are connected (e.g., by connection circuit 1 shown in Fig. 1) to display device 8, which may display the graph shown in Fig. 2, lor alternatively, a numeral result from the measurements taken by sensors 4.

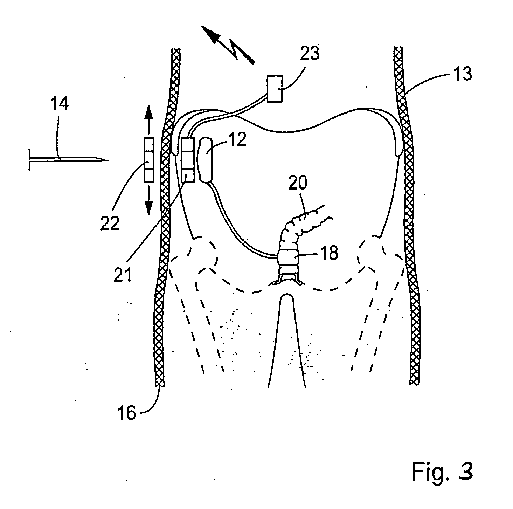

Fig. 3 shows an embodiment of the apparatus of the present invention for detecting an injection port 12 subcutaneously implanted in a patient 13 suffering from anal. incontinence to enable positioning of an injection needle 14 outside the patient's body for safe and accurate injection in the injection port 12. Injection port 12 is hydraulically connected to a hydraulically adjustable artificial sphincter 18 applied to the patient's rectum 20. The apparatus also includes

a magnetic detector 21 subcutaneously implanted in patient 13 at injection port 12 and an external separate magnetic device in the form of a ring-magnet 22 that emits a local magnetic field through patient's 13 skin 16. Ring-magnet 22 may be manually moved externally along the patient's 13 body to establish an injection position at the patient's skin where the local magnetic field emitted by magnetic device 22 is detected by the implanted magnetic detector 21. A sender 23 is implanted in patient 13 and sends information about the status of magnetic detector 21. Thus, when magnetic detector 21 detects the local magnetic field emitted by external ring magnet 22, sender 23 sends information confirming that magnetic device 22 is in the proper injection position for accurate positioning of the injection needle 14 outside the patient's body. When this injection position has been established, the injection needle14 can be placed in the same position to accurately insert the needle through patient's skin directly into injection port 12.

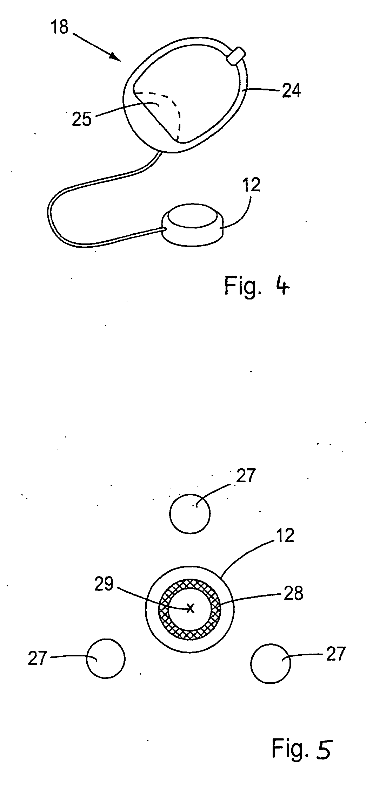

Fig. 4 shows an example of the artificial sphincter 18 shown in Figs. 3. Sphincter 18 includes a hydraulically adjustable constriction device 24 to be applied around the patient's rectum (not shown in Fig. 4). Constriction device 24 has a cavity 25 which can be inflated by supplying hydraulic fluid thereto, via implanted injection port 12, to close the rectum, and be deflated by withdrawing hydraulic fluid therefrom, to open the rectum. This type of constriction device may also be used as an artificial sphincter for treating patient's suffering from heartburn and reflux disease or urinary incontinence, when combined with the apparatus of the present invention. Furthermore, constriction device 24 may be used for forming an adjustable stoma opening in the stomach or esophagus of an obese patient to treat obesity or for restricting the penile exit blood flow to treat an impotent patient, when combined with the apparatus of the invention.

Fig. 5 shows an advantageous design of the embodiment shown in Fig. 3, in which the implanted magnetic detector 21 includes three symmetrically arranged Hall-elements 27 which are grouped around a central point in a triangular configuration. The magnetic device includes a ring-magnet 28 surrounding the centre 29 of the implanted injection port 12. When ring-magnet 28 is moved to a position in which Hall-elements 27 are placed symmetrically underneath and around ring-magnet 28, as illustrated in Fig. 5, magnetic detector 21 detects a maximum intensity of the magnetic field emitted by the ring-magnet 28, whereby the most accurate position where the injection needle 14 should be inserted into injection port 12 is established.

## Claims

1. An injection port detection apparatus, comprising:
a permanent ring-magnet (22) adapted to emit a local permanent magnetic field through the skin (16) of a patient from outside the patient's body, and
a magnetic detector (21) capable of being subcutaneously implanted in the patient at an injection port (12) implanted in the patient and adapted to detect the local permanent magnetic field emitted by the ring-magnet,
wherein the ring-magnet (22) is movable externally along the patient's body to establish an injection position at the patient's skin (16) in front of the implanted injection port where the local permanent magnetic field emitted by the ring-magnet is detected by the magnetic detector, whereby an injection needle can be placed in the established injection position, in order to insert the injection needle through the hole of the ring-magnet and through the patient's skin directly into the injection port substantially in the centre thereof.

2. An apparatus according to claim 1, wherein the magnetic detector (21) comprises a semiconductor circuit.

3. An apparatus according to claim 2, wherein the semiconductor circuit of the magnetic detector (21) comprises at least one Hall-element (27).

4. An apparatus according to claim 3, wherein the magnetic detector (21) comprises several Hall-elements (27) grouped around a central point in a triangular or square-configuration.

5. An apparatus according to any one of claims 1-4, further comprising a sender (23) implantable in the patient's body and capable of sending information about the magnetic detector (21) to outside the patient's body, as the magnetic detector detects the local permanent magnetic field emitted by the ring-magnet (22) from outside the patient's body.

6. An apparatus according to any one of claims 1-4, wherein the magnetic detector is adapted to emit a sound when detecting the local permanent magnetic field.

7. An artificial sphincter apparatus comprising:
- an artificial sphincter (18) capable of being implanted inside a patient's body and comprising a hydraulically operable constriction device (24) having a cavity (25) inflatable by supplying hydraulic fluid thereto,
- an injection port (12) capable of being subcutaneously implanted in the patient's body and hydraulically connected to the constriction device (24) for inflating and deflating the cavity (25) by supplying to and withdrawing from the cavity (25) hydraulic fluid via the injection port (12), and
- the injection port detection apparatus according to any of claims 1 to 6 for detecting the injection port (12) when implanted.

## Patentansprüche

1. Vorrichtung zur Detektion eines Injektionsports, umfassend:
einen permanenten Ringmagneten (22), der angepasst ist, ein lokales permanentes Magnetfeld durch die Haut (16) eines Patienten von außerhalb des Körpers des Patienten zu emittieren, und
einen Magnetdetektor (21), der imstande ist, subkutan in einen Patienten an einem Injektionsport (12), der in dem Patienten implantiert ist, implantiert zu werden und angepasst ist, das lokale permanente Magnetfeld, das von dem Ringmagneten emittiert wird, zu detektieren,
wobei der Ringmagnet (22) außen entlang des Körpers des Patienten bewegbar ist, um eine Injektionsstelle auf der Haut (16) des Patienten vor dem implantierten Injektionsport zu ermitteln, wo das lokale permanente Magnetfeld, das von dem Ringmagneten emittiert wird, von dem Magnetdetektor detektiert wird, wobei eine Injektionsnadel an der ermittelten Injektionsstelle platziert werden kann, um die Injektionsnadel durch das Loch des Ringmagneten und durch die Haut des Patienten direkt in den Injektionsport, im Wesentlichen in dessen Zentrum, einzuführen.

2. Vorrichtung nach Anspruch 1, wobei der Magnetdetektor (21) eine Halbleiterschaltung umfasst.

3. Vorrichtung nach Anspruch 2, wobei die Halbleiterschaltung des Magnetdetektors (21) zumindest ein Hall-Element (27) umfasst.

4. Vorrichtung nach Anspruch 3, wobei der Magnetdetektor (21) mehrere Hall-Elemente (27) umfasst, die um einen zentralen Punkt in einer Dreiecks- oder Vierecks-Konfiguration gruppiert sind.

5. Vorrichtung nach einem der Ansprüche 1 bis 4, weiter umfassend einen Sender (23), der in den Körper des Patienten implantierbar und imstande ist, Informationen über den Magnetdetektor (21) nach außerhalb des Körpers des Patienten zu senden, wenn der Magnetdetektor das lokale permanente Magnetfeld, das von dem Ringmagneten (22) von außerhalb des Körpers des Patienten emittiert wird, detektiert.

6. Eine Vorrichtung nach einem der Ansprüche 1 bis 4, wobei der Magnetdetektor angepasst ist, einen Ton auszusenden, wenn er das lokale permanente Magnetfeld detektiert.

7. Eine künstliche Schließmuskelvorrichtung, umfassend:
- einen künstlichen Schließmuskel (18), der imstande ist, in den Körper eines Patienten implantiert zu werden, und der eine hydraulisch betreibbare Verengungseinrichtung (24) umfasst, die eine Kavität (25) besitzt, die durch Zuführen von hydraulischem Fluid aufpumpbar ist,
- einen Injektionsport (12), der imstande ist, subkutan in den Körper des Patienten implantiert zu werden, und der hydraulisch mit der Verengungseinrichtung (24) verbunden ist, um die Kavität (25) durch Zuführen und Abführen von hydraulischem Fluid zu und aus der Kavität (25) über den Injektionsport (12) aufzupumpen und zu entleeren, und
- die Vorrichtung zur Detektion eines Injektionsports nach einem der Ansprüche 1 bis 6 zum Detektieren des Injektionsports (12), wenn dieser implantiert ist.

## Revendications

1. Appareil de détection d'orifice d'injection, comprenant :
un aimant annulaire permanent (22) conçu pour émettre un champ magnétique permanent local à travers la peau (16) d'un patient de l'extérieur du corps du patient, et
un détecteur magnétique (21) pouvant être implanté par voie sous-cutanée chez le patient au niveau d'un orifice (12) d'injection implanté chez le patient et conçu pour détecter le champ magnétique permanent local émis par l'aimant annulaire,
dans lequel l'aimant annulaire (22) peut être déplacé de l'extérieur le long du corps du patient pour établir une position d'injection au niveau de la peau (16) du patient en face de l'orifice d'injection implanté où le champ magnétique permanent local émis par l'aimant annulaire est détecté par le détecteur magnétique, une aiguille d'injection pouvant être placée dans la position d'injection établie, de façon à insérer l'aiguille d'injection à travers le trou de l'aimant annulaire et à travers la peau du patient directement dans l'orifice d'injection sensiblement au centre de celui-ci.

2. Appareil selon la revendication 1, dans lequel le détecteur magnétique (21) comprend un circuit semi-conducteur.

3. Appareil selon la revendication 2, dans lequel le circuit semi-conducteur du détecteur magnétique (21) comprend au moins un élément de Hall (27).

4. Appareil selon la revendication 3, dans lequel le détecteur magnétique (21) comprend plusieurs éléments de Hall (27) groupés autour d'un point central dans une configuration triangulaire ou carrée.

5. Appareil selon l'une quelconque des revendications 1 à 4, comprenant en outre un émetteur (23) implantable dans le corps du patient, et pouvant envoyer des informations relatives au détecteur magnétique (21) vers l'extérieur du corps du patient, lorsque le détecteur magnétique détecte le champ magnétique permanent local émis par l'aimant annulaire (22) de l'extérieur du corps du patient.

6. Appareil selon l'une quelconque des revendications 1 à 4, dans lequel le détecteur magnétique est conçu pour émettre un son quand il détecte le champ magnétique permanent local.

7. Appareil de sphincter artificiel comprenant :
un sphincter artificiel (18) pouvant être implanté à l'intérieur du corps d'un patient et comprenant un dispositif (24) de constriction à commande hydraulique ayant une cavité (25) gonflable par apport d'un fluide hydraulique à celui-ci,
un orifice d'injection (12) pouvant être implanté par voie sous-cutanée dans le corps d'un patient et relié hydrauliquement au dispositif (24) de constriction pour gonfler et dégonfler la cavité (25) en fournissant à la cavité (25) et en retirant de celle-ci un fluide hydraulique par le biais de l'orifice (12) d'injection, et
l'appareil de détection de l'orifice d'injection selon l'une quelconque des revendications 1 à 6 pour détecter l'orifice (12) d'injection quand il est implanté.
